# EUROPÄISCHE PATENTSCHRIFT

(11) **EP 1 440 065 B1**
(45) Veröffentlichungstag und Bekanntmachung des Hinweises auf die Patenterteilung: **06.10.2010**
(21) Anmeldenummer: 02782982.9
(22) Anmeldetag: 23.10.2002
(51) Int. Cl.: C07D 251/70, C07D 251/18

(54) **VERFAHREN ZUR HERSTELLUNG VON ALKOXYCARBONYLAMINO-TRIAZINEN**
METHOD FOR PREPARING ALKOXYCARBONYLAMINO-TRIAZINES
PROCEDE DE PREPARATION D'ALCOXYCARBONYLAMINO-TRIAZINES

(30) Priorität: 23.10.2001 DE 10151564; 25.04.2002 DE 10218617
(43) Veröffentlichungstag der Anmeldung: 28.07.2004
(73) Patentinhaber: BASF SE, 67056 Ludwigshafen (DE)
(72) Erfinder: SCHNEIDER, Jörg, 69469 Weinheim (DE); SCHERR, Günter, 67065 Ludwigshafen (DE); SCHUPP, Hans, 67549 Worms (DE); EICHFELDER, Andreas, 67133 Maxdorf (DE); ROBERT, Alain, 67150 Niederkirchen (DE); REIF, Martin, 67354 Römerberg (DE)
(86) Internationale Anmeldenummer: PCT/EP2002/011837
(87) Internationale Veröffentlichungsnummer: WO 2003/035628

(56) Entgegenhaltungen:
- EP-A- 0 624 577
- WO-A-00/29829
- US-B1- 6 204 382

## Beschreibung

Die vorliegende Erfindung betrifft ein neues Verfahren zur Herstellung von Alkoxycarbonylamino-triazinen durch Umsetzung von Di- oder Triaminotriazinen mit Dimethylcarbonat in Gegenwart eines Alkanols und eines Alkali- oder Erdalkalimethanolats als Base.

Aus der EP-A-624 577 ist die Herstellung von Alkoxycarbonylamino-triazinen durch Umsetzung von Triazinen, beispielsweise Melamin, mit Kohlensäureestern in Gegenwart einer Base bekannt. In der Regel wird dort Melamin mit einem Kohlensäureester, z.B. Dimethylcarbonat, in Gegenwart des dem Kohlensäureester zugrundeliegenden Alkanols, hier z.B. Methanol, und in Gegenwart eines Alkalialkanolats, basierend auf dem dem Kohlensäureester zugrundeliegenden Alkanol, hier z.B. Methanol, als Base zur Reaktion gebracht. Es wird weiterhin beschrieben, Melamin z.B. mit Dimethylcarbonat in Gegenwart eines höheren Alkohols, beispielsweise Butanol oder 2-Ethylhexanol, und des entsprechenden Natriumalkanolats, hier z.B. Natriumbutanolat oder Natrium-(2-ethylhexanolat) als Base umzusetzen.

Die dort beschriebene Verfahrensweise ist, wenn man andere Alkoxycarbonylamino-triazine als Methoxycarbonylamino-triazine oder wenn man Mischungen verschieden substituierter Alkoxycarbonylamino-triazine herstellen will, nachteilig. Man muss nämlich dann entweder von Dimethylcarbonat als einem wohlfeilen und im technischen Maßstab leicht verfügbaren Edukt abweichen und die Umsetzung mit höheren Dialkylcarbonaten, die in der Regel teuer und im technischen Maßstab nur schwer verfügbar sind, durchführen oder anstelle der ebenfalls wohlfeilen und im technischen Maßstab leicht verfügbaren Alkalimethanolate, insbesondere Natriummethanolat, als Base müssen Alkanolate von höheren Alkoholen zur Anwendung gelangen, die in der Regel im Vergleich zu den Methanolaten ebenfalls teuer und schwerer verfügbar sind.

Weiterhin ist die in der EP-A-624 577 beschriebene Aufarbeitung der Alkoxycarbonylamino-triazine aus den anfallenden Reaktionsmischungen nicht besonders vorteilhaft. In der Regel werden die Reaktionsmischungen angesäuert oder in eine saure Lösung übergeführt. Anschließend erfolgt eine Extraktion mit einem organischen Lösungsmittel. Die organischen Extrakte werden dann getrocknet und vom Lösungsmittel befreit. Alternativ wird nach dem Ansäuern ein Feststoff durch Filtration isoliert, der dann gewaschen und getrocknet wird.

Diese Methoden sind beider technischen Aufarbeitung von Reaktionsmischungen, die Alkoxycarbonylamino-triazine enthalten, nachteilig, da sie entweder der Einführung eines Extraktionsmittels in den Prozess oder eine kostenintensive Filtrationstechnik bedingen. Ferner ist in beiden Fällen eine Trocknung (Wasserentfernung) entweder der organischen Lösung mittels eines Trocknungsmittels oder des Feststoffes unter vermindertem Druck erforderlich.

Aufgabe der vorliegenden Erfindung war es nun, ein neues Verfahren zur Herstellung von Alkoxycarbonylamino-triazinen bereitzustellen, dass die genannten Nachteile nicht mehr aufweist und das gleichzeitig die Herstellung eines großen Spektrums von Mischungen gemischt funktionalisierter und/oder isomerer Alkoxycarbonylamino-triazine erlaubt.

Eine weitere Aufgabe der vorliegenden Erfindung war es, eine neue Aufarbeitungsmethode für Reaktionsmischungen, die bei der Herstellung von Alkoxycarbonylamino-triazinen anfallen, bereitzustellen. Dabei sollte auf eine kostenintensive Filtrationstechnik verzichtet und die Anzahl der chemischen Komponenten im Prozess nicht weiter erhöht werden.

Es wurde nun gefunden, dass die Herstellung von Alkoxycarbonylamino-triazinen der Formel I 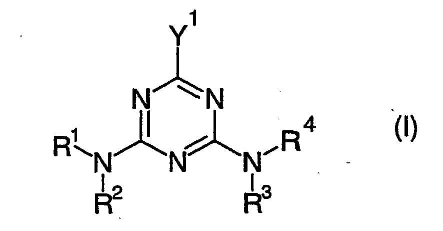 in der
- Y¹: Wasserstoff, C₁-C₄-Alkyl, gegebenenfalls durch C₁-C₄-Alkyl, C₁-C₄-Alkoxy oder Halogen substituiertes Phenyl oder einen Rest der Formel NR⁵R⁶ und
- R¹, R², R³, R⁴, R⁵ und R⁶: unabhängig voneinander jeweils Wasser- stoff oder einen Rest der Formel COOX oder X, worin X für C₁-C₁₃-Alkyl, dessen Kohlenstoffgerüst durch 1 oder 2 Sauer- stoffatome in Etherfunktion unterbrochen sein kann, steht, bedeuten,
mit der Maßgabe, dass mindestens einer der Reste R¹ bis R⁴, oder wenn Y¹ für NR⁵R⁶ steht, mindestens einer der Reste R¹ bis R⁶ Rest COOX bedeutet,
durch Umsetzung eines Triazins der Formel II 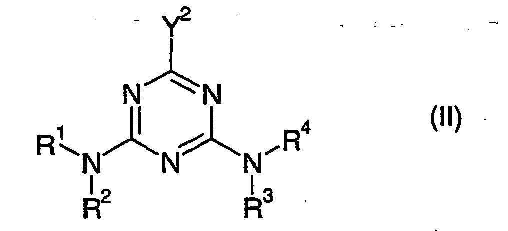 in der
- Y²: Wasserstoff, C₁-C₄-Alkyl, Amino oder gegebenenfalls durch C₁-C₄-Alkyl, C₁-C₄-Alkoxy oder Halogen substituiertes Phenyl bedeutet, mit der Maßgabe, dass in Formel II, wenn Y² nicht für Amino steht, mindestens einer der Reste R¹ bis R⁴ Wasser- stoff bedeutet, und
- R¹ bis R⁴: jeweils die obengenannte Bedeutung besitzen, mit Kohlensäureestern in Gegenwart eines Alkohols und einer Base vorteilhaft gelingt, wenn man das Triazin der Formel II mit Dimethylcarbonat und einem C₂-C₁₃-Alkanol, dessen Kohlenstoffgerüst durch 1 oder 2 Sauerstoffatome in Etherfunktion unterbrochen sein kann, in Gegenwart eines Alkali- oder Erdalkalimethanolats als Base umsetzt.

Alle in den hier aufgeführten Formeln enthaltenen Alkylreste können sowohl geradkettig als auch verzweigt sein.

Reste Y¹, Y² und X sind z.B. Methyl, Ethyl, Propyl, Isopropyl, Butyl, Isobutyl, sec-Butyl oder tert-Butyl.

Reste X sind weiterhin z.B. Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl, Undecyl, Dodecyl, Tridecyl, Isotridecyl, 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 4-Methoxybutyl, 2- oder 4-Ethoxybutyl, 3,6-Dioxaheptyl, 3,6-Dioxaoctyl, 3,7-Dioxaoctyl, 4,7-Dioxaoctyl, 2- oder 3-Butoxypropyl oder 2- oder 4-Butoxybutyl. (Die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmann's Encyclopedia of Industrial Chemistry, 5th Edition, Vol. A 1, Seiten 290 bis 293, sowie Vol. A 10, Seiten 284 und 285). Reste Y¹ und Y² sind weiterhin z.B. Phenyl, 2-, 3-, oder 4-Methylphenyl, 2-, 3-, oder 4-Ethylphenyl, 2,4-Dimethylphenyl, 2-, 3-oder 4-Methoxyphenyl, 2-, 3-, oder 4-Ethoxyphenyl, 2,4-Dimethoxyphenyl, 2-, 3- oder 4-Fluorphenyl oder 2-, 3- oder 4-Chlorphenyl.

Geeignete C₂-C₁₃-Alkanole, die im erfindungsgemäßen Verfahren zur Anwendung gelangen können, sind beispielsweise Ethanol, Propanol, Isopropanol, Butanol, Isobutanol, sec-Butanol, tert-Butanol, Pentanol, Isopentanol, Neopentanol, tert-Pentanol, Hexanol, 2-Methylpentanol, Heptanol, Octanol, 2-Ethylhexanol, Isooctanol, Nonanol, Isononanol, Decanol, Isodecanol, Undecanol, Dodecanol, Tridecanol, Isotridecanol, 2-Methoxyethanol, 2-Ethoxyethanol, 2-Propoxyethanol, 2-Butoxyethanol, 2- oder 3-Methoxypropanol, 2- oder 3-Ethoxypropanol, 2- oder 3-Propoxypropanol, 2- oder 4-Methoxybutanol, 2- oder 4-Ethoxybutanol, 3,6-Dioxaheptanol, 3,6-Dioxaoctanol, 3,7-Dioxaoctanol, 4,7-Dioxaoctanol, 2- oder 3-Butoxypropanol oder 2- oder 4-Butoxybutanol.

Bevorzugt ist die Verwendung von C₂-C₁₃-Alkanolen, wobei die Verwendung von C₂-C₇-Alkanolen besonders zu nennen ist.

Die im erfindungsgemäßen Verfahren verwendeten Alkohole können entweder einzeln oder auch als Mischungen untereinander zur Anwendung gelangen. Im letzteren Fall können die Anzahl der Mischungspartner sowie die Mischungsverhältnisse beliebig sein.

Geeignete Alkali- oder Erdalkalimethanolate, die erfindungsgemäß zur Anwendung gelangen können, sind z.B. Lithium-, Natrium-, Kalium-, Magnesium- oder Calciummethanolat. Die Verwendung von Alkalimethanolaten, insbesondere von Natriummethanolat ist bevorzugt.

Alkali- oder Erdalkalimethanolat kann entweder in festem Aggregatzustand oder in gelöster oder suspendierter Form zur Anwendung gelangen.

Bevorzugte Lösungsmittel/Verdünnungsmittel sind in diesem Fall insbesondere die oben näher bezeichneten Alkohole, allein oder als Mischung untereinander. Es können jedoch auch andere an sich bekannte und übliche inerte Verdünnungsmittel zur Anwendung gelangen.

Es ist weiterhin auch möglich, eine methanolische Lösung von Alkali- oder Erdalkalimethanolat in das erfindungsgemäße Verfahren einzubringen. Bei Durchführung dieser Variante ist der Gewichtsanteil an Methanol, bezogen auf das Gesamtgewicht aller im Verfahren verwendeter Alkohole (einschließlich Methanol) kleiner oder gleich 20 Gew.-%, vorzugsweise kleiner 15 Gew.-%.

Eine Verfahrensweise bei der die Umsetzung in Gegenwart eines Katalysators vorgenommen wird, ist ebenfalls möglich.

Beispielsweise können Phasentransferkatalysatoren der Art, wie sie z.B. in Ullmann's Encyclopedia of Industrial Chemistry, 5th Edition, Vol. A 19, Seiten 239 bis 248, beschrieben sind, verwendet werden.

Weitere Katalysatoren können Metallsalze oder -komplexe sein, vorzugsweise Oxide, Chalkogenate, Carbonate oder Halogenide der-Alkali-, Erdalkali- oder Übergangsmetalle. Zu nennen sind hier insbesondere beispielsweise Lithiumchlorid, Magnesiumchlorid oder Natriumcarbonat.

Im erfindungsgemäßen Verfahren kommen je Moläquivalent an substituierbaren Aminogruppen im Triazin der Formel II in der Regel 1 bis 50 mol, vorzugsweise 3 bis 30 mol, Alkanol zum Einsatz.

Unter substituierbaren Aminogruppen im erfindungsgemäßen Sinn sind die folgenden Gruppierungen zu verstehen: -NH₂ oder -NH-.

Weiterhin kommen im erfindungsgemäßen Verfahren je Moläquivalent an substituierbaren Aminogruppen im Triazin der Formel II in der Regel 0,1 bis 10 mol, vorzugsweise 1 bis 3 mol, Dimethylcarbonat zum Einsatz.

Weiterhin kommen im erfindungsgemäßen Verfahren je Moläquivalent an substituierbaren Aminogruppen im Triazin der Formel II in der Regel 0,1 bis 10 mol, vorzugsweise 1 bis 3 Moläquivalent, Alkali- oder Erdalkalimethanolat zum Einsatz.

Falls das erfindungsgemäße Verfahren in Gegenwart eines Katalysators durchgeführt wird, kommen im allgemeinen 10⁻¹⁰ bis 10 Gew.-%, vorzugsweise 10⁻³ bis 1 Gew.-%, Katalysator, jeweils bezogen auf das Gewicht des Triazins der Formel II, zur Anwendung.

Das erfindungsgemäße Verfahren wird im allgemeinen bei einer Temperatur von 20 bis 180°C, vorzugsweise 50 bis 100°C, durchgeführt.

Man arbeitet üblicherweise unter atmosphärischem Druck, wobei jedoch die Anwendung von erhöhtem Druck, in der Regel bis zu 8 bar, möglich ist.

Von besonderem Interesse ist die Verwendung von Triazinen der Formel II, in der Y² Amino bedeutet, als Edukt im erfindungsgemäßen Verfahren, wobei die Verwendung von Melamin (2,4,6-Triamino-1,3,5-triazin) ganz besonders hervorzuheben ist.

Von ganz besonderem Interesse ist die Herstellung von Alkoxycarbonylamino-triazinen der Formel III 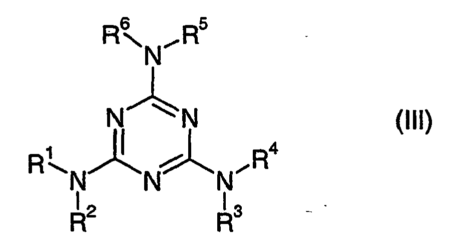 in der

R¹ bis R⁶ jeweils die obengenannte Bedeutung besitzen, mit der Maßgabe, dass drei dieser Reste jeweils Wasserstoff und die restlichen drei dieser Reste jeweils einen Rest der Formel COOX bedeuten, worin X die obengenannte Bedeutung besitzt, mittels des erfindungsgemäßen Verfahrens.

Obwohl unter Formel I auch solche Produkte fallen, die i) nur einen einzigen Rest COOX im Molekül aufweisen, worin X für Methyl steht, und/oder die ii) mehrere Reste COOX im Molekül aufweisen, worin X ausschließlich für Methyl steht, sind solche Produkte allein nicht als Zielprodukte des erfindungsgemäßen Verfahrens zu verstehen. Sie fallen allenfalls in Mischung mit anderen Produkten an, die a) nur einen einzigen Rest COOX im Molekül aufweisen, worin X verschieden von Methyl ist, und/oder die b) mehrere Reste COOX im Molekül aufweisen, worin mindestens ein Rest X verschieden von Methyl ist.

Vorteilhaft wird das erfindungsgemäße Verfahren so durchgeführt, dass man Triazin II und Alkanol vorlegt und dann in beliebiger Reihenfolge Alkali- oder Erdalkalimethanolat, in festem Zustand und/oder gelöst in Alkanol, und Dimethylcarbonat zudosiert, wobei die Dosierung von Alkali- oder Erdalkalimethanolat und Dimethylcarbonat vollständig vor Reaktionsbeginn oder teilweise vor Reaktionsbeginn und teilweise nach Reaktionsbeginn erfolgen kann. Durch Abdestillieren gewisser Mengen von Alkanol aus dem Reaktionsgemisch vor und/oder während der Reaktion kann man die Einstellung gewünschter Alkanolverhältnisse bewirken, wodurch das Produktspektrum gezielt beeinflusst werden kann.

Die Herstellung der erfindungsgemäßen Alkoxycarbonylaminotriazine kann dabei in verschiedenen Varianten (A-F) erfolgen.

In Variante A) werden Triazin II, Alkanol und das gelöste Alkali- oder Erdalkalimethanolat zusammengegeben. Anschließend wird bei erhöhter Temperatur, in der Regel 30 bis 85°C, Dimethylcarbonat hinzugegeben.

In Variante B) werden alle Komponenten vor Reaktionsbeginn vorgelegt.

In Variante C) werden Triazin II, Alkanol und Dimethylcarbonat vorgelegt und ein Teil des Alkali- oder Erdalkalimethanolats, das teilweise in gelöster und teilweise in festem Zustand vorliegt, vor Reaktionsbeginn und der Rest nach Reaktionsbeginn zudosiert.

In Variante D) werden Triazin II, Alkanol und Dimethylcarbonat vorgelegt und ein Teil des Alkali- oder Erdalkalimethanolats, das entweder in gelöster Form oder in festem Zustand vorliegt, vor Reaktionsbeginn und der Rest nach Reaktionsbeginn zudosiert.

In Variante E) werden vor oder nach Reaktionsbeginn Katalysatoren zugegeben.

In Variante F) werden vor oder nach Reaktionsbeginn unterschiedliche Alkalimethanolate zugegeben (z.B. Lithiummethanolat und Natriummethanolat).

Die Reaktionsführung kann auch derart erfolgen, dass Melamin während der Reaktion stufenweise oder kontinuierlich zudosiert wird.

Das erfindungsgemäße Verfahren kann in üblichen Reaktionsapparaturen, z.B. einem Kessel- oder Rohrreaktor, durchgeführt werden. Wenn man das neue Verfahren so durchführt, dass das Molverhältnis Triazin der Formel II:Alkanol möglichst hoch ist, ist die Verwendung von Apparaten mit Mischwirkung bei hochviskosen oder inhomogenen Reaktionsmischungen, z.B. Knetreaktoren, bevorzugt. Auch die Verwendung von selbstreinigenden Apparaten mit Mischwirkung ist möglich. Solche Apparate sind an sich bekannt und handelsüblich. Geeignete Reaktoren dieser Art sind z.B. der Kammerreaktor, der Kreislaufreaktor oder der Schneckenreaktor. Vorteilhaft findet die Aufarbeitung des resultierenden Reaktionsgemisches in Abwesenheit zusätzlicher Lösungsmittel statt.

Dazu wird die alkanolische Reaktionsmischung direkt mit Säure, entweder durch Zudosieren von Säure oder durch Überführung der Reaktionsmischung in eine geeignete Säure, in Kontakt gebracht. Die Säure kann dabei konzentriert zugegeben werden und die Zugabe von Wasser während oder nach der Zudosierung der Säure erfolgen. Insbesondere bei Verwendung von wässrigen oder hochkonzentrierten Säuren muss während der Dosierung eine geeignete Durchmischung gewährleistet werden. Zum Ansäuern des Reaktionsgemisches können alle üblichen und industriell verfügbaren organischen und anorganischen Säuren, gegebenenfalls auch in Mischung untereinander, in beliebiger Konzentration, vorzugsweise jedoch als 30 bis 85 gew.-%ige wässrige Lösungen, verwendet werden. Vorzugsweise verwendet man Mineralsäuren, deren Salze eine hohe Wasserlöslichkeit aufweisen, wie Salpetersäure, Schwefelsäure oder Phosphorsäure oder deren Mischungen, aber auch die Carbonsäure Ameisensäure ist hier zu nennen.

Nach der Zugabe von Säure zum Reaktionsgemisch bilden sich eine wässrige und eine alkanolische Phase, die von einander getrennt werden. Die Trennung der Phasen ist temperatur- und pH-Wert-abhängig, so dass die Zugabe von zusätzlichem Wasser bei einer Temperatur von 10 bis 70°C, bevorzugt von 15 bis 50°C, und bei einem pH-Wert von 0 bis 7, bevorzugt von 2 bis 4, erfolgt.

Die Zielprodukte resultieren direkt als 10 bis 80 gew.-%ige alkanolische Lösung. Durch anschließendes Einengen der alkanolischen Phase wird gleichzeitig mitgeschlepptes Wasser zum Teil azeotrop entfernt (z.B. bei Butanol), so dass weitere Trocknungsschritte, z.B. der Zusatz von Trocknungsmittel, nicht erforderlich sind.

Die Aufarbeitung des Reaktionsgemisches kann nach Neutralisation mit einer beliebigen Säure, selbstverständlich auch durch Extraktion, Waschen und/oder durch Filtration erfolgen.

Das neue Verfahren, das sowohl in kontinuierlicher wie auch in diskontinuierlicher Arbeitsweise vorgenommen werden kann, liefert die Zielprodukte in hoher Ausbeute und Reinheit.

Bei den mittels des erfindungsgemäßen Verfahrens erhältlichen Alkoxycarbonylamino-triazinen handelt es sich um wertvolle Lackrohstoffe.

Die folgenden Beispiele sollen die Erfindung näher erläutern.

Alle Reaktionen wurden vorzugsweise unter Feuchtigkeitsausschluss durchgeführt. Bei Alkylcarbonylamino-triazin-Gemischen ließen sich die einzelnen Komponenten mittels HPLC (20 µl Schleife; UV-Detektor (250 nm); 1 ml/min, Acetonitril : wässr. Kalium-dihydrogenphosphat (0,05 mol/1) =1:1; Säule Purospher-RP18e) trennen. Die Mengenangabe der Komponenten in den folgenden Beispielen erfolgt in Flächenprozent (A.-%). Die Stoffidentifikation erfolgte durch hochauflösende Massenspektrometrie, teilweise in Form einer direkten HPLC-MS-Kopplung oder mittels ¹H- und ¹³C-Kernresonanzspektroskopie.

### Beispiel 1

25 g (0,2 mol) Melamin, 1200 ml Butanol und 180,1g (1 mol) Natriummethanolat (30 gew.-%ig in Methanol) wurden bei einer Temperatur von 20°C vorgelegt. Anschließend wurde unter verminderten Druck (460 mbar) bis zu einer Destillatmenge von ca. 130 ml destilliert. Die Reaktionsmischung wurde auf ca. 90°C erhitzt und innerhalb einer Minute mit 59,5 g (0,66 mol) Dimethylcarbonat versetzt. Nach beendeter Zugabe wurde das inhomogene Reaktionsgemisch weitere 90 Minuten bei ca. 95°C gerührt. Nach Abkühlen der Mischung auf ca. 30°C wurden unter Rühren 210 g (1 mol) wässrige Salpetersäure (30 gew.-%ig) und 300 ml Wasser zugegeben. Die wässrige Phase wurde abgetrennt und die organische homogene Phase weitere 2 mal mit je 300 ml Wasser gewaschen. Durch Einengen der organischen Phase resultierte eine 50 gew.-%ige butanolische Produktlösung, die überwiegend 2,4,6-Tris(butoxycarbonylamino)-1,3,5-triazin, 2-Methoxycarbonylamino-4,6-bis(butoxycarbonylamino)-1,3,5-triazin und 2,4-Bis-(methoxycarbonylamino)- 6-butoxycarbonylamino-1,3,5-triazin enthielt (HPLC, ¹H-, ¹³C-NMR).

### Beispiel 2

25 g (0,2 mol) Melamin, 1872 ml Heptanol und 180,1g (1 mol) Natriummethanolat (30 gew.-%ig in Methanol) wurden bei einer Temperatur von 20°C vorgelegt. Anschließend wurde bis zu einer Destillatmenge von ca. 33 ml destilliert. Die Reaktionsmischung wurde bei ca. 90°C innerhalb einer Minute mit 59,5 g (0,66 mol) Dimethylcarbonat versetzt. Nach beendeter Zugabe wurde das inhomogene Reaktionsgemisch weitere 90 Minuten bei 100°C gerührt. Durch direkte Analyse der Reaktionsmischung ließen sich die Hauptkomponenten der Mischung identifizieren als 2,4,6-Tris-(heptyloxycarbonylamino)-1,3,5-triazin, 2-Methoxycarbonylamino-4,6-bis(heptyloxycarbonylamino)-1,3,5-triazin und 2,4-Bis(methoxycarbonylamino)-6-heptyloxycarbonylamino-1,3,5-triazin (HPLC, HPLC-MS).

### Beispiel 3

Beispiel 3 wurde analog Beispiel 1 durchgeführt, jedoch wurden anstelle von Butanol 774 ml Ethanol verwendet. Durch direkte Analyse der Reaktionsmischung ließen sich die Hauptkomponenten der Mischung identifizieren als 2,4,6-Tris(ethoxycarbonylamino)-1,3,5-triazin, 2-Methoxycarbonylamino-4,6-bis(ethoxycarbonylamino)-1,3,5-triazin und 2,4-Bis(methoxycarbonylamino)-6-ethoxycarbonylamino-1,3,5-triazin (HPLC, HPLC-MS).

### Beispiel 4

Beispiel 4 wurde analog Beispiel 1 durchgeführt, jedoch wurden anstelle von Butanol 1002 ml 2-Propanol verwendet. Durch direkte Analyse der Reaktionsmischung ließen sich die Hauptkomponenten der Mischung identifizieren als 2,4,6-Tris(2-propoxycarbonylamino)-1,3,5-triazin, 2-Methoxycarbonylamino-4,6-bis(2-propoxycarbonylamino)-1,3,5-triazin und 2,4-Bis(methoxycarbonylamino)-6-(2-propoxy)carbonylamino-1,3,5-triazin (HPLC, HPLC-MS).

### Beispiel 5

Beispiel 5 wurde analog dem Beispiel 1 durchgeführt, jedoch wurden anstelle von Natriummethanolat 385 g Lithiummethanolat (10 gew.-%ig in Methanol) verwendet. Durch direkte Analyse der Reaktionsmischung ließen sich die Hauptkomponenten der Mischung identifizieren als 2,4,6-Trisbutoxycarbonylamino)-1,3,5-triazin, 2-Methoxycarbonylamino-4,6-bis(butoxycarbonylamino)-1,3,5-triazin und 2,4-Bis(methoxycarbonylamino)-6-butoxycarbonylamino-1,3,5-triazin (HPLC, HPLC-MS).

### Beispiel 6

25 g (0,2 mol) Melamin, 300 ml Butanol, 115,8 g (0,64 mol) Natriummethanolat (30 gew.-%ig in Methanol), 19,3 g (0,36 mol) Natriummethanolat und 59,5 g (0,66 mol) Dimethylcarbonat wurden bei 20°C vorgelegt. Die Reaktionsmischung wurde auf 95°C erhitzt ca. 60 Minuten bei dieser Temperatur gerührt. Die Reaktionsmischung wurde aufgearbeitet, wie in Beispiel 1 erläutert, und enthielt überwiegend 2,4,6-Tris(butoxycarbonylamino)-1,3,5-triazin, 2-Methoxycarbonylamino-4,6-bis(butoxycarbonylamino)-1,3,5-triazin und 2,4-Bis(methoxycarbonylamino)-6-butoxycarbonylamino-1,3,5-triazin (HPLC).

### Beispiel 7

Beispiel 7 wurde analog Beispiel 6 jedoch bei einem Druck von ca. 2 bar und einer Reaktionstemperatur von 80°C durchgeführt. Die Reaktionsmischung wurde aufgearbeitet, wie in Beispiel 1 erläutert, und enthielt überwiegend 2,4,6-Tris(butoxycarbonylamino)-1,3,5-triazin, 2-Methoxycarbonylamino-4,6-bis(butoxycarbonylamino)-1,3,5-triazin und 2,4-Bis(methoxycarbonylamino)-6- butoxycarbonylamino-1,3,5-triazin enthielt (HPLC).

### Beispiel 8

50 g (0,4 mol) Melamin, 134 ml Butanol, 151,3 g (2,8 mol) Natriummethanolat (fest) und 144 g (1,6 mol) Dimethylcarbonat wurden bei einer Temperatur von 20°C in einen Knetreaktor (List-Reaktor) gefüllt. Anschließend wurde die Reaktionsmischung 1 Stunde bei 75°C geknetet. Durch direkte Analyse der Reaktionsmischung ließen sich die Hauptkomponenten der Mischung identifizieren als 2,4,6-Tris(methoxycarbonylamino)-1,3,5-triazin, 2-Butoxycarbonylamino-4,6-bis(methoxycarbonylamino)-1,3,5-triazin und 2,4-Bis(butoxycarbonylamino)-6-methoxycarbonylamino-1,3,5-triazin, 2,4,6-Tris(butoxycarbonylamino)-1,3,5-triazin, (HPLC, HPLC-MS).

### Beispiel 9

25,2 g (0,2 mol) Melamin, 1200 ml Butanol, 75,6 g (1,4 mol) Natriummethanolat (fest) und 72,06 g (0,8 mol) Dimethylcarbonat wurden bei einer Temperatur von 20°C vorgelegt. Anschließend wurde die Reaktionsmischung auf ca. 70°C erhitzt und weitere 90 Minuten bei ca. 70°C gerührt. Nach Abkühlen der Mischung auf ca. 30°C wurden unter Rühren 294 g (1,4 mol) wässrige Salpetersäure (30 gew.-%ig) und 300 ml Wasser zugegeben. Die wässrige Phase wurde abgetrennt und die organische homogene Phase weitere 2 mal mit je 300 ml Wasser gewaschen. Durch Einengen der organischen Phase resultierte eine 50 gew.-%ige butanolische Produktlösung, die überwiegend 2,4,6-Tris(butoxycarbonylamino)-1,3,5-triazin, 2-Methoxycarbonylamino-4,6-bis-(butoxycarbonylamino)-1,3,5-triazin und 2,4-Bis(methoxycarbonylamino)-6-butoxycarbonylamino-1,3,5-triazin enthielt (HPLC, ¹H-, ¹³C-NMR).

### Beispiel 10

Die Reaktion wurde analog Beispiel 9 durchgeführt, jedoch wurden anstelle von Salpetersäure nach Abkühlen der Mischung auf ca. 30°C unter Rühren 97 g (1 mol) wässrige Phosphorsäure (85 gew.-%ig) und 600 ml Wasser zugegeben. Die wässrige Phase wurde abgetrennt und die organische homogene Phase weitere 2 mal mit je 300 ml Wasser gewaschen. Durch Einengen der organischen Phase resultierte eine 50 gew.-%ige butanolische Produktlösung, die überwiegend 2,4,6-Tris(butoxycarbonylamino)-1,3,5-triazin, 2-Methoxycarbonylamino-4,6-bis(butoxycarbonylamino)-1,3,5-triazin und 2,4-Bis(methoxycarbonylamino)-6-butoxycarbonylamino-1,3,5-triazin enthielt (HPLC, ¹H-, ¹³C-NMR).

### Beispiel 11

Die Reaktion wurde analog Beispiel 9 und 10 durchgeführt, jedoch wurde während des Waschvorgangs der pH-Wert der Mischung durch Zugabe von Salpetersäure unter 3 gehalten. Nach weiterer Aufarbeitung analog dem Beispiel 10 resultierte eine 50 gew.-%ige butanolische Produktlösung, die überwiegend 2,4,6-Tris(butoxycarbonylamino)-1,3,5-triazin, 2-Methoxycarbonylamino-4,6-bis-(butoxycarbonylamino)-1,3,5-triazin und 2,4-Bis(methoxycarbonylamino)-6-butoxycarbonylamino-1,3,5-triazin enthielt (HPLC, ¹H-, ¹³C-NMR).

### Beispiel 12

Die Reaktion wurde analog Beispiel 9 durchgeführt, jedoch wurden nach Abkühlen der Mischung auf ca. 30°C anstelle von Salpetersäure unter Rühren 458 g (0,7 mol) wässrige Schwefelsäure (50 gew.-%ig) und 600 ml Wasser zugegeben. Die wässrige Phase wurde abgetrennt und die organische homogene Phase weitere 2 mal mit je 300 ml Wasser gewaschen. Durch Einengen der organischen Phase resultierte eine 50 gew.-%ige butanolische Produktlösung, die überwiegend 2,4,6-Tris(butoxycarbonylamino)-1,3,5-triazin, 2-Methoxycarbonylamino-4,6-bis(butoxycarbonylamino)-1,3,5-triazin und 2,4-Bis(methoxycarbonylamino)-6-butoxycarbonylamino-1,3,5-triazin enthielt (HPLC, ¹H-, ¹³C-NMR).

### Beispiel 13

29 g (0,23 mol) Melamin, 82,9 g (0,92 mol) Dimethylcarbonat und 87 g (1,61 mol) Natriummethanolat wurden in 1200 ml Butanol gegeben. Das Reaktionsgemisch wurde 3 Stunden bei 78°C gerührt. Nach Abkühlen auf Raumtemperatur wurden innerhalb von 1 Minute 338,2 g (286,6 ml) 30 gew.-%ige wässrige Salpetersäure zudosiert. Nach Abtrennung der wässrigen Phase wurde die Mischung 3 mal mit je 200 ml Wasser gewaschen. Destillatives Entfernen von Wasser und Butanol führte zu einer 50 gew.-%igen butanolischen Lösung, die als Hauptkomponenten 2,4,6-Tris(butoxycarbonylamino)-1,3,5-triazin, 2-Methoxycarbonylamino-4,6-bis(butoxycarbonylamino)-1,3,5-triazin und 2,4-Bis(methoxycarbonylamino)-6-butoxycarbonylamino-1,3,5-triazin enthielt (HPLC, ¹H-, ¹³C-NMR).

### Beispiel 14

Die Reaktion wurde analog Beispiel 13 durchgeführt, jedoch wurde das Melamin in 4 gleichen Portionen innerhalb von 1,5 Stunden zum vorgelegten Dimethylcarbonat, Natriummethanolat und Butanol dosiert. Die Aufarbeitung erfolgte ebenfalls wie in Beispiel 13 beschrieben. Es resultierte eine 50 gew.-%ige butanolische Lösung, die als Hauptkomponenten 2,4,6-Tris(butoxycarbonylamino)-1,3,5-triazin, 2-Methoxycarbonylamino-4,6-bis(butoxycarbonylamino)-1,3,5-triazin und 2,4-Bis(methoxycarbonylamino)-6-butoxycarbonylamino-1,3,5-triazin enthielt (HPLC, ¹H-, ¹³C-NMR).

### Beispiel 15

2,9 kg (23 mol) Melamin, 120 1 Butanol, 8,7 kg (161 mol) Natriummethanolat und 8,3 kg (92 mol) Dimethylcarbonat wurden zusammengegeben und unter Rühren innerhalb von einer Stunde auf 85°C erhitzt. Die Reaktionsmischung wurde weitere 2,5 Stunden bei 85°C gerührt und anschließend auf 35°C abgekühlt. Unter kräftigem Rühren wurden 33,8 kg (161 mol) 30 gew.-%ige wässrige Salpetersäure zugegeben und nach weiteren 15 Minuten die Rührung beendet. Nach erfolgter Phasenseparation wurde die wässrige Phase abgetrennt und verworfen. Die organische Phase wurde 3 mal mit je 20 l Wasser gewaschen. Anschließend wurde die organische Phase unter vermindertem Druck eingeengt. Die resultierende ca. 50 gew.-%ige Lösung enthielt überwiegend 2,4,6-Tris(butoxycarbonylamino)-1,3,5-triazin (19,5 A.-%), 2-Methoxycarbonylamino-4,6-bis(butoxycarbonylamino)-1,3,5-triazin (41,5 A.-%), 2,4-Bis(butoxycarbonylamino)-6-amino-1,3,5-triazin (2.8 A.-%), 2,4-Bis(methoxycarbonylamino)-6-butoxycarbonylamino-1,3,5-triazin (25.6 A.-%), 2-Methoxycarbonylamino-4-butoxycarbonylamino-6-amino-1,3,5-triazin (3.4 A.-%) sowie Tris(methoxycarbonylamino)-1,3,5-triazin (4,6 A.-%).

### Beispiel 16

Die Reaktion wurde analog Beispiel 15 durchgeführt, jedoch wurde die Mischung innerhalb einer Stunde auf 80°C erhitzt. Anschließend wurden unter verminderten Druck ca. 15 l Lösungsmittel innerhalb von 30 Minuten abdestilliert. Die Reaktionsmischung wurde weitere 2,5 Stunden bei 80°C gerührt und anschließend auf 35°C abgekühlt. Die Aufarbeitung erfolgt wie in Beispiel 15 beschrieben. Die resultierende ca. 50 gew.-%ige Lösung enthielt überwiegend 2,4,6-Tris(butoxycarbonylamino)-1,3,5-triazin (25.9 A.-%), 2-Methoxycarbonylamino-4,6-bis(butoxycarbonylamino)-1,3,5-triazin (44.6 A.-%), 2,4-Bis(butoxycarbonylamino)-6-amino-1,3,5-triazin (2.4 A.-%), 2,4-Bis(methoxycarbonylamino)-6-butoxycarbonylamino-1,3,5-triazin (21.0 A.-%), 2-Methoxycarbonylamino-4-butoxycarbonylamino-6-amino-1,3,5-triazin (3.3 A.-%) sowie Tris(methoxycarbonylamino)-1,3,5-triazin (2,0 A.-%).

### Beispiel 17

Die Reaktion wurde analog Beispiel 16 durchgeführt, jedoch wurde nach Abdestillieren von 15 l Lösungsmittel nur 2 Stunden bei 80°C gerührt anschließend wurden nochmals während 30 Minuten ca. 15 l Lösungsmittel abdestilliert, bevor die Reaktionsmischung auf 35°C abgekühlt wurde. Die Aufarbeitung erfolgte wie unter Bespiel 15 beschrieben. Die resultierende ca. 50 gew.-%ige Lösung enthielt überwiegend 2,4,6-Tris(butoxycarbonylamino)-1,3,5-triazin (28.1 A.-%), 2-Methoxycarbonylamino-4,6-bis(butoxycarbonylamino)-1,3,5-triazin (42.75 A.-%), 2,4-Bis(butoxycarbonylamino)-6-amino-1,3,5-triazin (3.0 A.-%), 2,4-Bis(methoxycarbonylamino)-6-butoxycarbonylamino-1,3,5-triazin (18.4 A.-%), 2-Methoxycarbonylamino-4-butoxycarbonylamino-6-amino-1,3,5-triazin (4,8 A.-%) sowie Tris(methoxycarbonylamino)-1,3,5-triazin (1.8 A.-%).

### Beispiel 18

120 l Butanol und 8,7 kg (161 mol) Natriummethanolat wurden auf 85°C unter verminderten Druck unter Abdestillieren von Lösungsmittel erhitzt. Anschließend wurde die resultierende Mischung unter Rühren mit 2,9 kg (23 mol) Melamin und 8,3 kg (92 mol) Dimethylcarbonat versetzt. Nach 2 Stunden Rühren bei 85°C wurde die Reaktionsmischung abgekühlt und mit 39,45 kg (80,5 mol) 20 gew.-%iger wässriger Schwefelsäure und unter kräftigem Rühren versetzt. Die weitere Aufarbeitung erfolgte wie unter Beispiel 15 beschrieben. Die resultierende ca. 50 gew.-%ige Lösung enthielt die gleichen Hauptkomponenten wie in Beispiel 15 (HPLC-MS). Der Gehalt an Tris(butoxycarbonylamino)-1,3,5-triazin betrug 36 A.-% (HPLC).

### Beispiel 19

Die Reaktion wurde analog Beispiel 18 durchgeführt, jedoch wurde während der Reaktion bei 85°C kontinuierlich ein Lösungsmittelstrom abgeführt (Destillation). Die Aufarbeitung der Reaktionsmischung erfolgte wie in Beispiel 15 beschrieben. Die 50 gew.-%ige Produktlösung enthielt überwiegend Tris(butoxycarbonylamino)-1,3,5- triazin, dessen Gehalt 69 A.-% betrug (HPLC).

### Beispiel 20

Die Reaktion wurde analog Beispiel 19 durchgeführt, jedoch wurde während der Reaktion das durch Destillation abgeführte Lösungsmittel teilweise durch Butanol ersetzt. Die Aufarbeitung erfolgte analog Beispiel 15. Vollständiges Einengen der Reaktionsmischung ergab einen Feststoff der überwiegend aus Tris(butoxycarbonylamino)-1,3,5-triazin (98 A.-%) (HPLC) bestand. Das Produkt ließ sich aus Acetonitril/n-Hexan (Diffusion) umkristallisieren. Anal.: Ber.: C 50,69, H 7,1, 0 22,5, N 19,71; Gef.: C 49,5, H 7,3, 0 23,1, N 18,6

## Patentansprüche

1. Verfahren zur Herstellung von Alkoxycarbonylamino-triazinen der Formel I 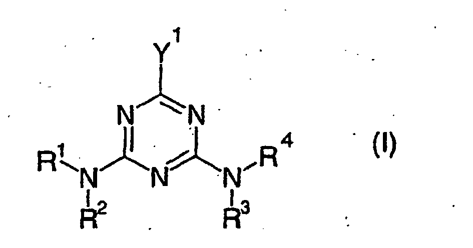 in der
Y¹ Wasserstoff, C₁-C₄-Alkyl, gegebenenfalls durch C₁-C₄-Alkyl, C₁-C₄-Alkoxy oder Halogen substituiertes Phenyl oder einen Rest der Formel NR⁵R⁶ und
R¹, R², R³, R⁴, R⁵ und R⁶ unabhängig voneinander jeweils Wasserstoff oder einen Rest der Formel COOX oder X, worin X für C₁-C₁₃-Alkyl, dessen Kohlenstoffgerüst durch 1 oder 2 Sauerstoffatome in Etherfunktion unterbrochen sein kann, steht, bedeuten;
mit der Maßgabe, dass in Formel I mindestens einer der Reste R¹ bis R⁴, oder wenn Y¹ für NR⁵R⁶ steht, mindestens einer der Reste R¹ bis R⁶ den Rest COOX bedeutet,
durch Umsetzung eines Triazins der Formel II 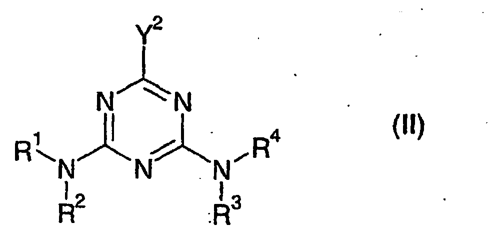 in der
Y² Wasserstoff, C₁-C₄Alkyl, Amino oder-gegebenenfalls durch C₁-C₄-Alkyl, C₁-C₄-Alkoxy oder Halogen substituiertes Phenyl bedeutet, mit der Massgabe, dass in Formel II, wenn Y² nicht für Amino steht, mindestens einer der Reste R¹ bis R⁴ Wasserstoff bedeutet, und
R¹ bis R⁴ jeweils die obengenannte Bedeutung besitzen,
mit Kohlensäureestern in Gegenwart eines Alkohols und
einer Base, **dadurch gekennzeichnet, dass** man das Triazin der Formel II mit Dimethylcarbonat und einem C₂-C₁₃-Alkanol, dessen Kohlenstoffgerüst durch 1 oder 2 Sauerstoffatome in Etherfunktion unterbrochen sein kann, in Gegenwart eines Alkali- oder Erdalkalimethanolats als Base umsetzt.

2. Verfahren nach Anspruch 1, **dadurch gekennzeichnet, dass** man ein C₂-C₁₃-Alkanol verwendet.

3. Verfahren nach Anspruch 1, **dadurch gekennzeichnet, dass** man ein Alkalimethanolat als Base verwendet.

4. Verfahren nach Anspruch 1, **dadurch gekennzeichnet, dass** man die Umsetzung bei einer Temperatur von 20 bis 180°C durchführt.

5. Verfahren nach Anspruch 1, **dadurch gekennzeichnet, dass** man die Umsetzung mit 1 bis 50 mol Alkanol, jeweils bezogen auf ein Moläquivalent an substituierbaren Aminogruppen im Triazin der Formel II, vornimmt.

6. Verfahren nach Anspruch 1, **dadurch gekennzeichnet, dass** man die Umsetzung mit 0,1 bis 10 mol Dimethylcarbonat, jeweils bezogen auf ein Moläquivalent an substituierbaren Aminogruppen im Triazin der Formel II, vornimmt.

7. Verfahren nach Anspruch 1, **dadurch gekennzeichnet, dass** man die Umsetzung mit 0,1 bis 10 Moläquivalent Alkali- oder Erdalkalimethanolat, jeweils bezogen auf ein Moläquivalent an substituierbaren Aminogruppen im Triazin der Formel II, vornimmt.

8. Verfahren nach Anspruch 1, **dadurch gekennzeichnet, dass** man Triazin II und Alkanol vorlegt und dann in beliebiger Reihenfolge Alkali- oder Erdalkalimethanolat, in festem Zustand und/oder gelöst in Alkanol, und Dimethylcarbonat zudosiert, wobei die Dosierung von Alkali- oder Erdalkalimethanolat und Dimethylcarbonat vollständig vor Reaktionsbeginn oder teilweise vor Reaktionsbeginn und teilweise nach Reaktionsbeginn erfolgen kann.

## Claims

1. A process for preparing alkoxycarbonylaminotriazines of the formula I 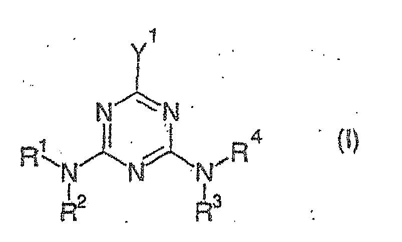 Where
Y¹ is hydrogen, C₁-C₄-alkyl, phenyl unsubstituted or substituted by C₁-C₄-alkyl, C₁-C₄-alkoxy or halogen, or is a radical of the formula NR⁵R⁶, and
R¹, R², R³, R⁴, R⁵ and R⁶ independently of one another are each hydrogen or a radical of the formula COOX or X, where X is C₁-C₁₃-alkyl, whose carbon skeleton may be interrupted by 1 or 2 oxygen atoms in ether function,
with the proviso that in formula I at least one of the radicals R¹ to R⁴ or, if Y¹ is NR⁵R⁶, at least one of the radicals R¹ to R⁶ is COOX,
by reacting a triazine of the formula II 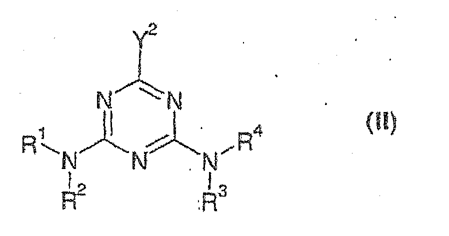 where
Y² is hydrogen, C₁-C₄-alkyl, amino or phenyl unsubstituted or substituted by C₁-C₄-alkyl, C₁- C₄-alkoxy or halogen, with the proviso that in formula II, if Y² is not amino, at least one of the radicals R¹ to R⁴ is hydrogen, and
R¹ to R⁴ are each as defined above,
with carbonic esters in the presence of an alcohol and a base, which comprises reacting said triazine of the formula II with dimethyl carbonate and a C₂-C₁₃-alkanol whose carbon skeleton may be interrupted by 1 or 2 oxygen atoms in ether function, in the presence of an alkali metal methoxide or alkaline earth metal methoxide as said base.

2. A process as claimed in claim 1, wherein a C₂-C₁₃-alkanol is used.

3. A process as claimed in claim 1, wherein an alkali metal methoxide is used as said base.

4. A process as claimed in claim 1, wherein the reaction is conducted at a temperature from 20 to 180°C.

5. A process as claimed in claim 1, wherein the reaction is performed with from 1 to 50 mol of alkanol, based in each case on one mole equivalent of substitutable amino groups in said triazine of the formula II.

6. A process as claimed in claim 1, wherein the reaction is performed with from 0.1 to 10 mol of dimethyl carbonate, based in each case on one mole equivalent of substitutable amino groups in said triazine of the formula II.

7. A process as claimed in claim 1, wherein the reaction is performed with from 0.1 to 10 mol equivalent of alkali metal methoxide or alkaline earth metal methoxide, based in each case on one mole equivalent of substitutable amino groups in said triazine of the formula II.

8. A process as claimed in claim 1, wherein triazine II and alkanol are introduced initially and then in any order alkali metal or alkaline earth metal methoxide, in solid sate and/or in solution in alkanol, and dimethyl carbonate are metered in, it being possible for the metering of alkali metal or alkaline earth metal methoxide and dimethyl carbonate to take place completely before the beginning of reaction or partly before the beginning of reaction and partly after the beginning of reaction.

## Revendications

1. Procédé pour la préparation d'alcoxycarbonylamino-triazines de formule I 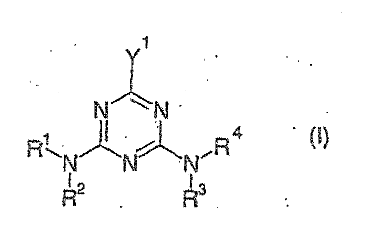 dans laquelle
Y¹ représente un atome d'hydrogène, un radical alkyle en C₁-C₄, un radical phényle éventuellement substitué par alkyle en C₁-C₄, alcoxy en C₁-C₄ ou halogène, ou un radical de formule NR⁵R⁶ et
R¹, R², R³, R⁴, R⁵ et R⁶ représentent chacun indépendamment les uns des autres, un atome d'hydrogène ou un radical de formule COOX ou X, dans lequel X représente un groupe alkyle en C₁-C₁₃, dont le squelette carboné peut être interrompu par 1 ou 2 atomes d'oxygène en fonction éther,
étant entendu que dans la formule I au moins l'un des radicaux R¹ à R⁴, ou, lorsque Y¹ représente NR⁵R⁶, au moins l'un des radicaux R¹ à R⁶, représente le radical COOX,
par mise en réaction d'une triazine de formule II 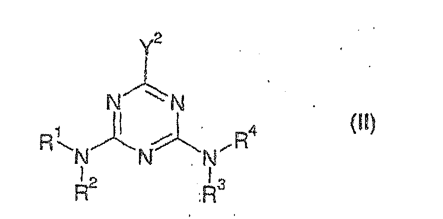 dans laquelle
Y² représente un atome d'hydrogène, un radical alkyle en C₁-C₄, un groupe amino ou un radical phényle éventuellement substitué par alkyle en C₁-C₄, alcoxy en C₁-C₄ ou halogène, étant entendu que dans la formule II, lorsque Y² ne représente pas un groupe amino, au moins l'un des radicaux R¹ à R⁴ représente un atome d'hydrogène, et
R¹ à R⁴ ont chacun la signification donnée ci-dessus,
avec des esters d'acide carbonique en présence d'un alcool et d'une base, **caractérisé en ce qu'**on fait réagir la triazine de formule II avec du carbonate de diméthyle et un alcanol en C₂-C₁₃, dont le squelette carboné peut être interrompu par 1 ou 2 atomes d'oxygène en fonction éther, en présence d'un méthanolate de métal alcalin ou alcalino-terreux en tant que base.

2. Procédé selon la revendication 1, **caractérisé en ce qu'**on utilise un alcanol en C₂-C₁₃.

3. Procédé selon la revendication 1, **caractérisé en ce qu'**on utilise comme base un méthanolate de métal alcalin.

4. Procédé selon la revendication 1, **caractérisé en ce qu'**on effectue la réaction à une température de 20 à 180 °C.

5. Procédé selon la revendication 1, **caractérisé en ce qu'**on effectue la réaction avec 1 à 50 moles d'alcanol, chaque fois par rapport à un équivalent molaire de groupes amino substituables dans la triazine de formule II.

6. Procédé selon la revendication 1, **caractérisé en ce qu'**on effectue la réaction avec 0,1 à 10 moles de carbonate de diméthyle, chaque fois par rapport à un équivalent molaire de groupes amino substituables dans la triazine de formule II.

7. Procédé selon la revendication 1, **caractérisé en ce qu'**on effectue la réaction avec 0,1 à 10 équivalents molaires d'un méthanolate de métal alcalin ou alcalino-terreux, chaque fois par rapport à un équivalent molaire de groupes amino substituables dans la triazine de formule II.

8. Procédé selon la revendication 1, **caractérisé en ce qu'**on dispose au préalable la triazine II et l'alcanol et on ajoute ensuite par addition dosée, en un ordre quelconque, un méthanolate de métal alcalin ou alcalino-terreux, à l'état solide et/ou dissous dans un alcanol, et du carbonate de diméthyle, l'addition dosée du méthanolate de métal alcalin ou alcalino-terreux et du carbonate de diméthyle pouvant s'effectuer entièrement avant le début de la réaction ou partiellement avant le début de la réaction et partiellement après le début de la réaction.
